(19) Europäisches Patentamt
European Patent Office
Office européen des brevets

(11) **EP 4 725 936 A1**

(12) **EUROPEAN PATENT APPLICATION**
published in accordance with Art. 153(4) EPC

(43) Date of publication:
**15.04.2026 Bulletin 2026/16**

(21) Application number: **24819415.1**

(22) Date of filing: **07.06.2024**

(51) International Patent Classification (IPC):
*C07C 68/08* (2006.01)    *C07B 61/00* (2006.01)
*C07C 68/06* (2020.01)    *C07C 68/065* (2020.01)
*C07C 69/96* (2006.01)

(52) Cooperative Patent Classification (CPC):
(C-Sets available)
**C07C 68/08; C07B 61/00; C07C 68/06**    (Cont.)

(86) International application number:
**PCT/JP2024/020877**

(87) International publication number:
**WO 2024/253188 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.06.2023 JP 2023094932**
**31.05.2024 PCT/JP2024/020121**

(71) Applicant: **ASAHI KASEI KABUSHIKI KAISHA**
**Tokyo 100-0006 (JP)**

(72) Inventor: **ENOMOTO, Miyako**
**Tokyo 100-0006 (JP)**

(74) Representative: **Strehl & Partner mbB**
**Maximilianstrasse 54**
**80538 München (DE)**

(54) **METHOD FOR PRODUCING CARBONATE HAVING ETHYL GROUP, AND APPARATUS FOR PRODUCING CARBONATE HAVING ETHYL GROUP**

(57)    A method for producing a carbonate having an ethyl group, the method comprising: a reaction step of subjecting dimethyl carbonate and ethanol to transesterification reaction in a reactor in the presence of a catalyst containing an alkali metal compound to obtain a reaction composition, a distillation step of distilling the reaction composition in a distillation column, an adsorption step of adsorbing a catalyst in the reaction composition by an adsorbent before the distillation step or during the distillation step.

[Figure 1]

(52) Cooperative Patent Classification (CPC): (Cont.)

C-Sets
**C07C 68/06, C07C 69/96;**
**C07C 68/08, C07C 69/96**

## Description

### Technical Field

The present invention relates to a method for producing a carbonate having an ethyl group and an apparatus for producing a carbonate having an ethyl group.

### Background Art

Carbonates having an ethyl group, such as ethyl methyl carbonate (EMC) and diethyl carbonate (DEC), are used as the organic solvent for battery electrolyte solutions. These carbonate compounds are known to be obtained from the transesterification reaction of dimethyl carbonate (DMC) and ethanol (EtOH). In the aforementioned transesterification reaction, an alkali metal compound such as an alkoxide compound of an alkali metal is used as the catalyst because it exhibits excellent activity (see Patent Document 1 and Patent Document 2).

### List of Prior Art Documents

### Patent Document

Patent Document 1: International Publication No. WO 2022/114592

Patent Document 2: International Publication No. WO 2022/114576

### Summary of Invention

### Problems to be Solved by Invention

Upon production of a carbonate having an ethyl group, an alkali metal compound is used as the catalyst for transesterification reaction. As the catalyst, an alkali metal compound such as sodium methoxide is used.
However, the alkali metal compound has a low solubility in an organic solvent, and for example, has properties of having a low solubility in DMC, EMC, and DEC, and the precipitation of the alkali metal compound may cause process troubles.
Regarding this, Patent Document 1 suggests a method for supplying an alkali metal compound in a state of being dissolved in dimethylsulfoxide. However, dimethylsulfoxide has a low solubility in the alkali metal compound as small as less than 1% by mass, and impurities are produced when raw materials, the alkali metal compound, and dimethylsulfoxide are mixed and heated.
Although Patent Document 2 discloses a method for removing insoluble matter by providing a filtration apparatus in a step subsequent to the reaction step, the filtration apparatus has problems in that it requires cleaning work and maintenance work, it causes blockage in piping for supplying materials to the filtration apparatus, and the like, and has further problems in that solid wastes derived from catalysts are strongly alkaline and water prohibitive, have high risks, and are hardly disposed.
An object of the present invention is to provide a method for producing a carbonate having an ethyl group in which the production of a precipitate can be suppressed in the distillation step and the distillation step can be continuously operated for a long term, and an apparatus for producing a carbonate having an ethyl group.

### Means for Solving Problems

The present inventors have found that the aforementioned problems can be solved by removing the catalyst in the reaction composition using an adsorbent before the distillation step.
The present invention encompasses the following embodiments.

<1> A method for producing a carbonate having an ethyl group, the method comprising:

a reaction step of subjecting dimethyl carbonate and ethanol to transesterification reaction in a reactor in the presence of a catalyst containing an alkali metal compound to obtain a reaction composition; and

a distillation step of distilling the reaction composition in a distillation column,

wherein the method comprises an adsorption step of adsorbing the alkali metal compound in the reaction composition by an adsorbent before the distillation step or during the distillation step.

<2> The method for producing the carbonate having the ethyl group according to <1>, wherein an insoluble matter concentration in the reaction composition in the reaction step is set to 50 ppm by mass or less.

<3> The method for producing the carbonate having the ethyl group according to <1> or <2>, wherein an alkali metal concentration in the reaction composition treated in the adsorption step is 500 ppb by mass or less.

<4> The method for producing the carbonate having the ethyl group according to any one of <1> to <3>, wherein the adsorbent in the adsorption step is a solid adsorbent.

<5> The method for producing the carbonate having the ethyl group according to any one of <1> to <4>, wherein the adsorbent in the adsorption step is an acidic cation exchange resin.

<6> The method for producing the carbonate having the ethyl group according to <5>, wherein the acidic cation exchange resin comprises a sulfonic acid group or a carboxylic acid group.

<7> The method for producing the carbonate having the ethyl group according to any one of <1> to <6>, wherein a concentration of the catalyst supplied to the reactor in the reaction step satisfies a condition of the expression (1):

[alkali metal concentration in the reaction composition (mol ppm)] $\leq$ 0.14 $\times$ [reaction temperature (°C)] $\times$ [alcohol concentration in reaction liquid (% by mol)] - 230      expression (1).

<8> The method for producing the carbonate having the ethyl group according to any one of <1> to <7>, further comprising a filtration step of filtering the reaction composition after the adsorption step.

<9> The method for producing the carbonate having the ethyl group according to any one of <1> to <8>, wherein a reaction temperature in the reaction step is 30°C or more and 110°C or less.

<10> The method for producing the carbonate having the ethyl group according to any one of <1> to <9>, wherein the reactor used in the reaction step is a stirred tank reactor or a plug flow reactor.

<11> The method for producing the carbonate having the ethyl group according to any one of <1> to <10>, wherein an adsorption column filled with the adsorbent is used in the adsorption step, and a superficial velocity of the adsorption column is 0.05 to 15 m/h.

<12> The method for producing the carbonate having the ethyl group according to any one of <1> to <11>, wherein a relationship between a flow rate of the adsorption column and an amount of the adsorbent filled, SV = [flow rate m3/h]/[amount of the adsorbent filled $m^3$-R] is 0.02 or more and 30 or less in the adsorption step.

<13> The method for producing the carbonate having the ethyl group according to any one of <1> to <12>, wherein the distillation step comprises at least one selected from the group consisting of an unreacted raw material removal step, a low-boiling point compound removal step, a high-purity ethyl methyl carbonate purification step, and a high-purity diethyl carbonate purification step.

<14> The method for producing the carbonate having the ethyl group according to <13>, wherein the distillation step comprises at least three selected from the group.

<15> The method for producing the carbonate having the ethyl group according to any one of <1> to <14>, wherein the distillation step comprises:

the unreacted raw material removal step,

the low-boiling point compound removal step, and

the high-purity ethyl methyl carbonate purification step or the high-purity diethyl carbonate purification step.

<16> An apparatus for producing a carbonate having an ethyl group, the apparatus comprising:

a reaction apparatus for subjecting dimethyl carbonate and ethanol to transesterification reaction in a reactor in the presence of a catalyst containing an alkali metal compound to obtain a reaction composition;

a catalyst adsorption apparatus for adsorbing the catalyst from the reaction composition by an adsorbent; and

a distillation apparatus for distilling the reaction composition.

<17> The apparatus for producing the carbonate having the ethyl group according to <16>, wherein the adsorbent is a solid adsorbent.

<18> The apparatus for producing the carbonate having the ethyl group according to <16> or <17>, wherein the adsorbent is an acidic cation exchange resin.

<19> The apparatus for producing the carbonate having the ethyl group according to <18>, wherein the acidic cation exchange resin comprises a sulfonic acid group or a carboxylic acid group.

<20> The apparatus for producing the carbonate having the ethyl group according to any one of <16> to <19>, wherein the catalyst adsorption apparatus is an adsorption column filled with the adsorbent.

<21> The apparatus for producing the carbonate having the ethyl group according to any one of <16> to <20>, further comprising a filter for filtering the reaction composition downstream the catalyst adsorption apparatus.

<22> The apparatus for producing the carbonate having the ethyl group according to any one of <16> to <21>, wherein the reaction apparatus is a stirred tank reactor or a plug flow reactor.

<23> The apparatus for producing the carbonate having the ethyl group according to any one of <16> to <22>, wherein the distillation apparatus comprises at least one selected from the group consisting of an unreacted raw material removal distillation column, a low-boiling point compound removal distillation column, a high-purity ethyl methyl carbonate purification distillation column, and a high-purity diethyl carbonate purification distillation column.

<24> The apparatus for producing the carbonate having the ethyl group according to <23>, wherein the distillation apparatus contains at least three selected from the group.

<25> The apparatus for producing the carbonate having the ethyl group according to any one of <16> to <24>, wherein the distillation apparatus comprises:

the unreacted raw material removal distillation column,

the low-boiling point compound removal distillation column, and

the high-purity ethyl methyl carbonate purification distillation column or the high-purity diethyl carbonate purification distillation column.

**Advantages of Invention**

The present invention can provide a method for producing a carbonate having an ethyl group in which the production of a precipitate can be suppressed in the distillation step and the distillation step can be continuously operated for a long term, and an apparatus for producing a carbonate having an ethyl group.

**Brief Description of Drawings**

[Figure 1] Figure 1 is a block diagram of the apparatus for producing a carbonate according to the present embodiment.

[Figure 2] Figure 2 is a schematic configuration diagram of an apparatus A for producing a carbonate according to the present embodiment.

[Figure 3] Figure 3 is a schematic configuration diagram of an apparatus B for producing a carbonate according to the present embodiment.

[Figure 4] Figure 4 is a schematic configuration diagram of an apparatus C for producing a carbonate according to the present embodiment.

[Figure 5] Figure 5 is a schematic configuration diagram of an apparatus for producing a carbonate according to the present embodiment.

[Figure 6] Figure 6 is a schematic configuration diagram of an apparatus for producing a carbonate according to the present embodiment.

[Figure 7] Figure 7 is a schematic configuration diagram of an apparatus for producing a carbonate according to a comparative example.

**Mode for Carrying Out Invention**

Hereinafter, the embodiment of the present invention (hereinafter, also referred to as "the present embodiment") will be described in detail with reference to the drawings as needed; however, the present invention is not limited to these, and can be variously modified without departing from the gist thereof. Unless otherwise specified, the positional relationship in the embodiment shown in the drawings, such as up, down, left, and right, is based on the positional relationship shown in the drawings. Further, the dimensional ratios in the drawings are not limited to the illustrated ratios.

The method for producing a carbonate having an ethyl group according to the present embodiment includes:

a reaction step of subjecting dimethyl carbonate and ethanol to transesterification reaction in a reactor in the presence of a catalyst containing an alkali metal compound to obtain a reaction composition; and

a distillation step of distilling the reaction composition in a distillation column,

wherein the method includes an adsorption step of adsorbing a catalyst in the reaction composition by an adsorbent before the distillation step or during the distillation step.

The above structure enables to provide the method for producing a carbonate having an ethyl group in which the production of a precipitate can be suppressed in the distillation step and the distillation step can be continuously operated for a long term.

The method for producing a carbonate having an ethyl group according to the present embodiment includes the reaction step and the distillation step, and includes the adsorption step before the distillation step or during the distillation step. The production method according to the present embodiment may have a raw material supply step of supplying a raw material containing dimethyl carbonate and ethanol to the reactor before the reaction step. In addition, the production method according to the present embodiment may have a reaction composition extraction step of extracting the reaction composition from the reactor after the reaction step.

The method for producing a carbonate having an ethyl group according to the present embodiment may be a continuous reaction by including a raw material supply step of continuously supplying a raw material containing dimethyl carbonate and ethanol to the reactor, and a reaction composition extraction step of continuously extracting the reaction composition from the reactor. The continuous reaction causes no blockage in the reactor and enables a high conversion rate of the raw material to be achieved.

<Raw Material Supply Step>

In the raw material supply step, the raw material is supplied to the reactor. The supply of the raw material may be continuously carried out.

The raw material contains dimethyl carbonate (hereinafter, simply referred to as "DMC") and ethanol (hereinafter, also referred to as "EtOH"). The raw material may contain a catalyst containing an alkali metal compound.

Examples of the alkali metal compound used as the catalyst include sodium hydroxide, potassium hydroxide, and an alkoxide of an alkali metal. Examples of the alkoxide of an alkali metal include lithium methoxide, lithium ethoxide, sodium methoxide, sodium methoxide, sodium ethoxide, potassium methoxide, and potassium ethoxide. Among these, the alkali metal compound is preferably a sodium-containing compound, more preferably sodium methoxide or sodium ethoxide, and still more preferably sodium methoxide, because the activity is high and thus economical operation with a low catalyst concentration is possible.

<Reaction Step>

In the reaction step, dimethyl carbonate and ethanol are subjected to transesterification reaction in a reactor in the presence of a catalyst containing an alkali metal compound to obtain a reaction composition containing a carbonate having an ethyl group. That is, the methoxy group in dimethyl carbonate is subjected to transesterification reaction with ethanol, so that a carbonate having an ethyl group is obtained. Examples of the carbonate having an ethyl group include ethyl methyl carbonate and diethyl carbonate.

(Reactor)

Examples of the reactor include, but are not particularly limited to, a stirred tank reactor, a reaction distillation column, and a plug flow reactor.

The concentration of the alkali metal compound in the reaction step is preferably 30,000 ppm by mass or less, more preferably 1,000 ppm by mass or less, and still more preferably 10 ppm by mass to 500 ppm by mass, based on the total amount of the reaction composition.

The concentration of the catalyst supplied to the reactor in the reaction step preferably satisfies the condition of the expression (1). By setting the concentration to the range, the amount of insoluble matter in the reaction composition can be reduced.

[Alkali metal concentration in the reaction composition (mol ppm)] $\leq$ 0.14 $\times$ [reaction temperature (°C)] $\times$ [alcohol concentration in reaction liquid (% by mol)] - 230          expression (1).

The alkali metal concentration in the reaction composition (mol ppm) is the molarity of the alkali metal based on the total molar amount of five components of methanol, ethanol, dimethyl carbonate, ethyl methyl carbonate, and diethyl carbonate which are the raw materials and reactants in the reaction composition.

The alcohol concentration in reaction liquid (% by mol) is the total molarity of methanol and ethanol based on the total molar amount of five components of methanol, ethanol, dimethyl carbonate, ethyl methyl carbonate, and diethyl carbonate which are the raw materials in the materials supplied and reactants.

[Concentration of Insoluble Matter in Reaction Composition]

The insoluble matter concentration in the reaction composition in the reaction step is preferably set to 50 ppm by mass or less.

When the insoluble matter concentration in the reaction composition in the reaction step is 50 ppm by mass or less, the adsorption step can be continuously operated for a long term without requiring the filtration step. Since a catalyst containing an alkali metal compound has a low solubility in an organic solvent such as dimethyl carbonate, insoluble matter is likely to be generated in the reaction composition. When the adsorption step is conducted in a state where the reaction composition contains a large amount of insoluble matter, clogging is likely to occur in an apparatus such as an adsorption column. Since the solubility in a solvent differs depending on the kind of alkali metal compound, an alkali metal compound having a high solubility in the solvent and having a high catalytic activity in a small amount used is selected in a preferred aspect, from the viewpoint of sufficiently reducing the catalyst concentration by adsorption without providing a filtration step for removing the catalyst. Specifically, a carbonate of an alkali metal has a low solubility in the solvent, whereas an alkoxide salt tends to exhibit solubility in a catalyst concentration satisfying the expression (1) and has a high catalytic activity. Thus, from the viewpoint of sufficiently reducing the catalyst concentration in the adsorption step, an alkoxide salt having a catalyst concentration satisfying the expression (1) is preferably used as the catalyst in the reaction step.

The insoluble matter concentration in the reaction composition treated in the adsorption step in the reaction step is 50 ppm by mass or less, more preferably 30 ppm by mass or less, and still more preferably 1 ppm by mass or less.

The method for measuring the concentration of insoluble matter in the reaction composition is the method described in Examples.

The reaction temperature in the reaction step is preferably 30°C to 110°C, more preferably 35°C to 100°C, and still more preferably 40°C to 90°C.

The pressure in the reaction step may be, for example, ordinary pressure to 1000 kPaG.

<Extraction Step>

In the extraction step, the reaction composition is extracted from the reactor. The extraction of the reaction composition may be continuously carried out. The extraction rate of the reaction composition is preferably set so that the following reactor residence time be 15 minutes or more and 2 hours or less.

[Reactor residence time (h)] = [liquid capacity in the reactor $(m^3)$]/[extraction rate $(m^3/h)$].

<Adsorption Step>

The production method according to the present embodiment includes an adsorption step of adsorbing the alkali metal compound in the reaction composition by an adsorbent before the distillation step or during the distillation step. By setting the concentration of the catalyst supplied to the reactor in the reaction step to the range satisfying the condition of the expression (1), the catalyst tends to be easily removed in the adsorption step. Examples of the alkali metal compound include the alkali metal compound contained in the catalyst, and a salt obtained by reacting the alkali metal compound with a weakly acidic substance or the like. Examples of the weakly acidic substance include carboxylic acid. Examples of the carboxylic acid include carboxylic acid having 2 to 30 carbon atoms, and more specific examples thereof include acetic acid, propionic acid, butanoic acid, hexanoic acid, and oleic acid.

(Adsorbent)

The adsorbent used in the adsorption step is preferably a solid adsorbent. By using a solid adsorbent, the catalyst can be removed from the liquid phase of the reaction composition.

Examples of the adsorbent include an acidic cation exchange resin, synthetic zeolite, a silica-alumina adsorbent, and a special inorganic adsorbent. Among these adsorbents, an acidic cation exchange resin is preferable due to a high selectivity and economical efficiency to the alkali metal.

The acidic cation exchange resin preferably has a sulfonic acid group or a carboxylic acid group.

Examples of the acidic cation exchange resin include commercial products such as Amberlite 200CT manufactured by ORGANO CORPORATION, Amberlite IRC76 manufactured by ORGANO CORPORATION, Amberlite 8400 manufactured by ORGANO CORPORATION, and Relite TM WK60L manufactured by Mitsubishi Chemical Corporation.

In the adsorption step, an adsorption column filled with an adsorbent may be used.

In the case of using an adsorption column, the superficial velocity of the adsorption column is preferably 0.05 to 15 m/h, more preferably 0.15 to 12 m/h, and still more preferably 0.3 to 10 m/h.

In the adsorption step, the relationship between the flow rate of the adsorption column and the amount of the adsorbent filled, SV = [flow rate of the adsorption column $(m^3/h)$]/[amount of the adsorbent filled $(m^3\text{-}R)$] is preferably 0.02 or more and 30 or less, more preferably 0.1 or more and 10 or less, and still more preferably 0.5 or more and 5 or less. By setting SV to 30 or less while setting the amount of the catalyst used in the reaction step to a preferred range, the concentration of the alkali metal compound in the reaction composition after the adsorption step is easily removed in a preferred range. The lower limit value of SV is set to optimize the amount of the adsorbent filled from an economical viewpoint.

The concentration of the alkali metal compound in the reaction composition after the adsorption step is preferably 5000 ppb by mass or less. To adjust the concentration of the alkali metal compound after the adsorption step to a preferred range, the adsorption apparatus is preferably operated so that the adsorption capacity is ensured, while the concentration of the catalyst supplied to the reactor in the reaction step is set to satisfy the condition of the expression (1) and the alkali metal concentration in the reaction composition that flows into the adsorption apparatus is thereby set to a range capable of being sufficiently treated in the adsorption step. Specifically, when the adsorption apparatus is an adsorption column, the superficial velocity is preferably set to 0.05 to 15 m/h, and SV is preferably set to 30 or less, more preferably 10 or less, and still more preferably 5 or less. When the alkali metal compound concentration in the reaction composition after the adsorption step is 500 ppb by mass or less, ethyl methyl carbonate is reacted in the distillation step, so that the yield of ethyl methyl carbonate can be prevented from being reduced. The alkali metal compound concentration is more preferably 150 ppb by mass or less, and still more preferably 100 ppb by mass or less.

When the distillation step is carried out by treatment using distillation apparatuses such as a plurality of distillation columns, the adsorption step is preferably carried out before the first distillation. Further, the adsorption step may be carried out immediately before distillation using each distillation apparatus.

The insoluble matter concentration in the reaction composition treated in the adsorption step is preferably 50 ppm by mass or less, more preferably 30 ppm by mass or less, and still more preferably 1 ppm by mass or less. The method for measuring the concentration of insoluble matter in the reaction composition is the method described in Examples.

Regarding the method for adjusting the concentration of insoluble matter in the reaction composition to the aforementioned range, the alkali metal compound can be dissolved in the reaction composition by adjusting the conditions to satisfy the condition of the expression (1) in the aforementioned reaction step or by adding a solvent in which the solubility of the alkali metal compound is high.

<Solid Removal Step>

From the viewpoint of removing a powdered adsorbent due to the wear and the like of the adsorbent that may be generated in the adsorption step, the production method according to the present embodiment preferably includes a solid removal step of removing a solid in the reaction composition after the reaction step and the adsorption step.

Examples of the removal of the solid in the reaction composition include a method using filtration.

<Distillation Step>

In the distillation step according to the present embodiment, the reaction composition is distilled in the distillation column. In the distillation step, a fraction containing 99.9% by mass or more of the carbonate having an ethyl group is preferably separated.

More specifically, the distillation step preferably includes at least one selected from the group consisting of an unreacted raw material removal step, a low-boiling point compound removal step, a high-purity ethyl methyl carbonate purification step, and a high-purity diethyl carbonate purification step, and more preferably includes at least three selected from the above group.

In the unreacted raw material removal step, for example, dimethyl carbonate and ethanol that are unreacted substances of the transesterification reaction in the reaction step are distilled off. Dimethylcarbonate and ethanol obtained in the step may be recycled in the reaction step.

In the low-boiling point compound removal step, for example, a purified product subjected to treatment such as the aforementioned unreacted raw material removal step is further distilled to increase the purity of carbonates having an ethyl group such as ethyl methyl carbonate and diethyl carbonate that are target compounds.

In the high-purity ethyl methyl carbonate purification step and the high-purity diethyl carbonate purification step, further distillation is carried out to obtain each of ethyl methyl carbonate and diethyl carbonate in a high purity enough to withstand electron material applications such as an electrolyte.

The distillation step preferably includes:

the unreacted raw material removal step,
the low-boiling point compound removal step, and
the high-purity ethyl methyl carbonate purification step or the high-purity diethyl carbonate purification step.

The carbonate having an ethyl group that is the target compound in the production method according to the present embodiment is ethyl methyl carbonate or dimethyl carbonate, and preferably ethyl methyl carbonate. These carbonates having an ethyl group are used, for example, as the electrolyte solution for lithium-ion secondary batteries.

As described above, the production method according to the present embodiment enables to provide the method for producing a carbonate having an ethyl group in which the production of the precipitate can be suppressed in the distillation step and the distillation step can be continuously operated for a long term.

[Apparatus for Producing Carbonate Having Ethyl Group]

Hereinafter, the apparatus for producing a carbonate having an ethyl group with which the production method according to the present embodiment is conducted (hereinafter, simply referred to as "the apparatus for producing a carbonate") will be described.

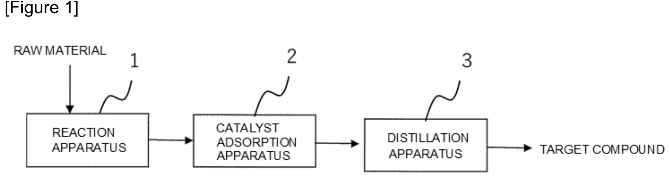

Figure 1 is a block diagram of the apparatus for producing a carbonate according to the present embodiment. The apparatus for producing a carbonate according to the present embodiment has a reaction apparatus 1, a catalyst adsorption apparatus 2, and a distillation apparatus 3. The production apparatus supplies a raw material to the reaction apparatus 1, and sequentially carries out the treatment, so that the reaction step, the adsorption step, and the distillation step are carried out in the order presented.

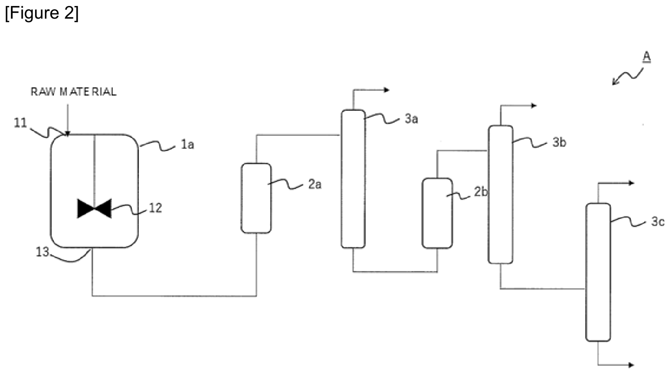

Figure 2 is a schematic configuration diagram of an apparatus A for producing a carbonate according to the present embodiment. The apparatus A for producing a carbonate has a stirred tank reactor 1a, a catalyst adsorption apparatus 2a, an unreacted raw material removal distillation column 3a, a catalyst adsorption apparatus 2b, a low-boiling point compound removal distillation column 3b, and a high-purity ethyl methyl carbonate purification distillation column 3c.

A raw material supply part 11 is provided at the stirred tank reactor 1a, and a raw material is supplied. Here, the raw material is the aforementioned raw material, which contains dimethyl carbonate and ethanol and may contain a catalyst containing an alkali metal compound. The raw material supply part may be independently provided for every raw material. The stirred tank reactor 1a has a stirring apparatus 12. An extraction part 13 is provided at the lower part of the stirred tank reactor 1a, and the reaction composition is extracted from the extraction part 13.

In the reaction composition extracted from the extraction part 13, the catalyst containing the alkali metal compound in the reaction composition is removed by the catalyst adsorption apparatus 2a.

Thereafter, the reaction composition is transferred to the unreacted raw material removal distillation column 3a, and unreacted substances such as dimethyl carbonate and ethanol are removed. The composition from which unreacted raw materials are distilled off is extracted from the column bottom of the unreacted raw material removal distillation column 3a.

In the composition subjected to the distillation treatment in the unreacted raw material removal distillation column 3a, the catalyst containing the alkali metal compound in the reaction composition is removed again by the catalyst adsorption apparatus 2b. The alkali metal compound in the composition is concentrated by distillation or the alkali metal compound is easily precipitated due to the change in the composition of the composition. Thus, by removing the catalyst again by the catalyst adsorption apparatus 2b, the generation of the precipitate in the subsequent distillation step can be suppressed.

The composition subjected to the treatment in the catalyst adsorption apparatus 2b is transferred to the low-boiling point compound removal distillation column 3b, and a low-boiling point compound is further removed. The composition from which the low-boiling point compound is distilled off is extracted from the column bottom of the low-boiling point compound removal distillation column 3b. In the low-boiling point compound removal step, the purity of carbonates having an ethyl group such as ethyl methyl carbonate and diethyl carbonate that are target compounds is increased.

The composition subjected to the treatment in the low-boiling point compound removal distillation column 3b is transferred to the high-purity ethyl methyl carbonate purification distillation column 3c to further increase the purity of ethyl methyl carbonate.

The high-purity ethyl methyl carbonate purification distillation column 3c may be the high-purity diethyl carbonate purification distillation column, according to the purpose.

As described above, according to the production apparatus A, the raw material supply step, the reaction step, the extraction step, the adsorption step, the unreacted raw material removal step, the adsorption step, the low-boiling point compound removal step, and the high-purity ethyl methyl carbonate purification step are carried out in the order presented.

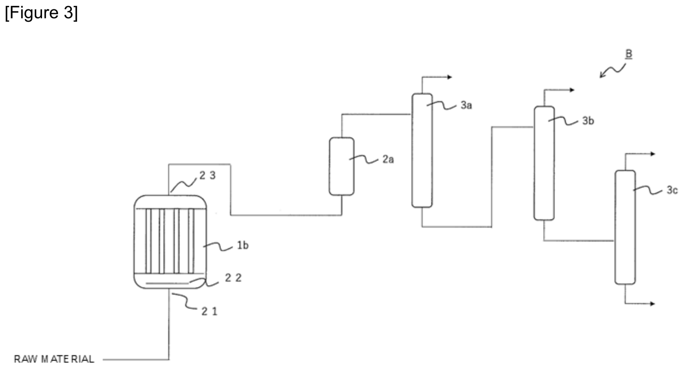

Figure 3 is a schematic configuration diagram of an apparatus B for producing a carbonate according to the present embodiment. The apparatus B for producing a carbonate has a plug flow reactor 1b, a catalyst adsorption apparatus 2a, an unreacted raw material removal distillation column 3a, a catalyst adsorption apparatus 2b, a low-boiling point compound removal distillation column 3b, and a high-purity ethyl methyl carbonate purification distillation column 3c. The portions common to those in the production apparatus A are denoted with the same reference signs, and the detailed description thereof is omitted.

A raw material supply part 21 is provided at the plug flow reactor 1b, and a raw material is supplied. Here, the raw material is the aforementioned raw material, which contains dimethyl carbonate and ethanol and may contain a catalyst containing an alkali metal compound. In the plug flow reactor 1b, the reaction is proceeded by a plug flow (piston flow) method. The raw material supply part may be independently provided for every raw material. When the raw material supply part is independently provided, the plug flow reactor 1b has a dispersion plate 22 inside thereof. An extraction part 23 is provided on the plug flow reactor 1b, and the reaction composition is extracted from the extraction part 23.

As described above, according to the production apparatus B, the raw material supply step, the reaction step, the extraction step, the adsorption step, the unreacted reaction raw material removal step, the low-boiling point compound removal step, and the high-purity ethyl methyl carbonate purification step are carried out in the order presented.

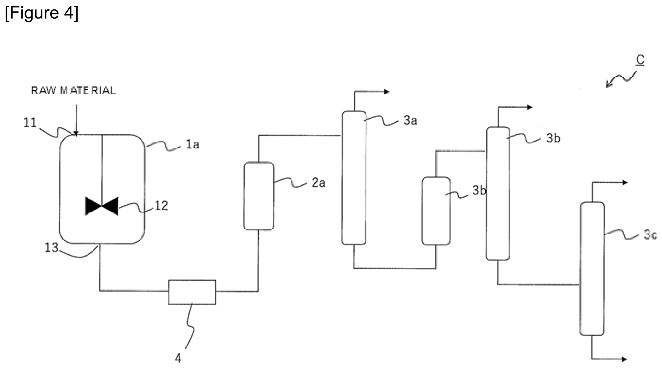

Figure 4 is a schematic configuration diagram of an apparatus C for producing a carbonate according to the present embodiment. The apparatus C for producing a carbonate has a stirred tank reactor 1a, a filter 4, a catalyst adsorption apparatus 2a, an unreacted raw material removal distillation column 3a, a catalyst adsorption apparatus 2b, a low-boiling point compound removal distillation column 3b, and a high-purity ethyl methyl carbonate purification distillation column 3c. The portions common to those in the production apparatus A are denoted with the same reference signs, and the detailed description thereof is omitted.

Since the apparatus for producing a carbonate C has the filter 4, the insoluble matter in the reaction composition is removed. Consequently, the catalyst adsorption apparatus 2a can be prevented from clogging and operated for a longer term.

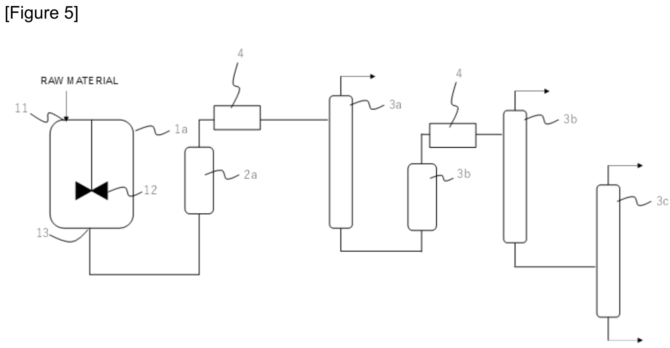

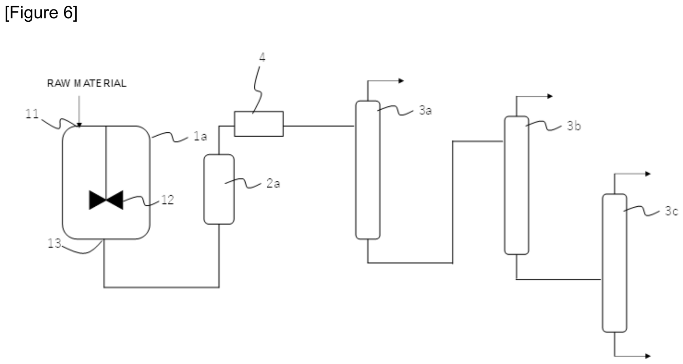

The apparatuses described in Figure 5 and Figure 6 are different from the apparatus described in Figure 4 in that the filter 4 is provided downstream the catalyst adsorption apparatus 2a or 3b. When the filter is provided downstream the catalyst adsorption apparatus, the adsorbent such as an ion exchange resin accommodated in the catalyst adsorption apparatus flowed out can be captured by the filter. As the filter material of the filter, the same filter material as the apparatus described in Figure 4 can be used. Since the metal compound as the catalyst is designed so as to be collected by the catalyst adsorption apparatus, a filter for capturing the metal compound is basically not required. However, a design as a measure for preventing trouble such that the adsorbent itself flows out from influencing the distillation step is effective in terms of industrial operation for a long term.

As described above, according to the production apparatus C, the raw material supply step, the reaction step, the filtration step, the extraction step, the adsorption step, the unreacted raw material removal step, the adsorption step, the low-boiling point compound removal step, and the high-purity ethyl methyl carbonate purification step are carried out in the order presented.

**Examples**

Hereinafter, the present embodiments will be described in more detail with reference to Examples, but the present invention is not limited to the following Examples.

[ICP Inductively Coupled Plasma Atomic Emission Spectroscopy]

Measurement was carried out using an ICP inductively coupled plasma atomic emission spectrometer " Agilent 5800 ICP-OES" manufactured by Agilent Technologies. The Na concentration in a sample can be determined by dissolving a weighed sample in nitric acid after being concentrated to dryness to prepare a liquid for measurement, and measuring the Na content of the obtained liquid for measurement by the above apparatus.

[Concentration of Insoluble Matter in Reaction Composition]

10,000 g of a reaction composition was extracted, and the reaction composition was filtered with a filter made of polytetrafluoroethylene (PTFE) having a pore size of 10 $\mu$m under a reduced pressure of -0.069 MPaG within 90 minutes after extraction. The mass of the filtered substance after completion of the filtration was measured, and the following equation was used as the concentration of insoluble matter in the reaction composition.

(Concentration of insoluble matter in a reaction composition [ppm by mass]) = (mass of filtered substance [g]) ÷ (filtered sample mass [g]) $\times$ 1,000,000

[Example 1]

Using the apparatus A for producing a carbonate having an ethyl group having a structure illustrated in Figure 2, carbonates having an ethyl group are produced as follows.

DMC, EtOH, and a 28% by mass solution of sodium methoxide in methanol are respectively supplied to a stirred tank reactor 1a having an internal diameter of 2,000 mm, a height of 3,000 mm, and a capacity of 6 m$^3$ at a flow rate of 970 kg/h, 290 kg/h, and 225 g/h, and reacted under conditions of 60°C and 103 kPaA, and 1,260 kg/h of a reaction product is extracted from the stirred tank reactor 1a to obtain a reaction composition containing carbonates having an ethyl group. The concentration of sodium methoxide as the catalyst in the reaction composition is 50 ppm by mass, and is 68 mol ppm as the alkali metal concentration. With respect to the amount of each component in the reaction composition, MeOH is 150 kg/h, EtOH is 74 kg/h, DMC is 581 kg/h, EMC is 411 kg/h, and DEC is 44 kg/h. The insoluble matter concentration in the reaction composition extracted from the stirred tank reactor 1a is 1 ppm by mass or less. The value of "0.14 $\times$ [reaction temperature (°C)] $\times$ [alcohol concentration in the reaction liquid (% by mol)] - 230" in the expression (1) is 80.

The reaction composition is supplied to a column filled with an ion exchange resin containing a sulfonic acid group as an exchange group (internal diameter: 800 mm, filling height: 1,600 mm) to adsorb and remove the catalyst (adsorption step (1)). At this time, the superficial velocity of the reaction composition in the adsorption step is 2.7 m/h and SV is 1.7 m3/m3-R. The analyzed Na concentration in the reaction product at the ion exchange resin column outlet is 3 ppb by mass.

The ion exchange resin column outlet liquid is supplied to the distillation step. The configuration of the distillation step is a configuration in which the column bottom outlet liquid of an unreacted raw material removal column as the first distillation column is supplied to a column similar to the above column filled with the ion exchange resin (adsorption step (2)), and is then supplied to a low-boiling point compound removal column as the second distillation column, and the column bottom outlet liquid of the second distillation column is supplied to a high-purity EMC purification column as the third distillation column.

As a result that the similar catalyst adsorption column is installed at the first distillation column bottom outlet, no blockage is caused over the entire production process, and continuous operation for 1,000 h or more can be conducted. The yield of ethyl methyl carbonate in the distillation step is 98% or more and 100% or less, and the yield of diethyl carbonate is also 98% or more and 100% or less.

[Example 2]

Using the apparatus B for producing a carbonate having an ethyl group having a structure illustrated in Figure 3, carbonates having an ethyl group are produced as follows.

DMC, EtOH, and a 20% by mass solution of sodium ethoxide in ethanol are respectively supplied to a plug flow reactor 1b with six tubes having an internal diameter of 80 mm and a length of 10,000 mm (capacity: 0.3 m$^3$) at a flow rate of 156 kg/h, 70 kg/h, and 89 g/h, and reacted under the conditions of 70°C and 103 kPaA, and 226 kg/h of a reaction composition is extracted from the plug flow reactor 1b to obtain the reaction composition containing carbonates having an ethyl group. The concentration of sodium ethoxide as the catalyst in the reaction composition is 79 ppm by mass, and is 80 mol ppm as the alkali metal concentration. With respect to the amount of each component in the reaction composition, MeOH was 32 kg/h, EtOH was 24 kg/h, DMC was kg/h, EMC was 80 kg/h, and DEC was 14 kg/h. The insoluble matter concentration in the reaction composition extracted from the plug flow reactor 1b is 1 ppm by mass or less. The value of "0.14 × [reaction temperature (°C)] × [alcohol concentration in the reaction liquid (% by mol)] - 230" in the expression (1) is 228.

The reaction composition is supplied to a column filled with an ion exchange resin containing a carboxylic acid group as an exchange group (internal diameter: 800 mm, filling height: 1,600 mm) to adsorb and remove the catalyst (adsorption step (1)). At this time, the superficial velocity of the reaction composition in the adsorption step is 0.05 m/h and SV is 0.03 m$^3$/m$^3$-R. The analyzed Na concentration in the reaction product at the ion exchange resin column outlet is 1 ppb by mass.

The ion exchange resin column outlet liquid is supplied to the distillation step. The configuration of the distillation step is a configuration in which the column bottom outlet liquid of an unreacted raw material removal column as the first distillation column is supplied to a low-boiling point compound removal column as the second distillation column, and the column bottom outlet liquid of the second distillation column is supplied to a high-purity EMC purification column as the third distillation column. No blockage is caused over the entire production process, and continuous operation for 1,000 h or more is possible. The yield of ethyl methyl carbonate in the distillation step is 98% or more and 100% or less, and the yield of diethyl carbonate is also 98% or more and 100% or less.

[Example 3]

Using the apparatus C for producing a carbonate having an ethyl group having a structure illustrated in Figure 4, carbonates having an ethyl group are produced as follows.

DMC, EtOH, and a 28% by mass solution of sodium methoxide in methanol are respectively supplied to a stirred tank reactor 1a having an internal diameter of 2,000 mm, a height of 3,000 mm, and a capacity of 6 m$^3$ at a flow rate of 970 kg/h, 290 kg/h, and 1,100 g/h, and reacted under conditions of 80°C and 200 kPaA, and 1,261 kg/h of a reaction product is extracted from the stirred tank reactor 1a to obtain a reaction composition containing carbonates having an ethyl group. The concentration of sodium methoxide as the catalyst in the reaction composition is 224 ppm by mass, and is 334 mol ppm as the alkali metal concentration. With respect to the amount of each component in the reaction composition, MeOH is 154 kg/h, EtOH is 70 kg/h, DMC is 587 kg/h, EMC is 389 kg/h, and DEC is 61 kg/h. The reaction composition is supplied to a filter, the insoluble matter concentration in the reaction composition before filtration is 83 ppm by mass, and the insoluble matter in the reaction product after filtration is 1 ppm by mass or less. The value of "0.14 × [reaction temperature (°C)] × [alcohol concentration in the reaction liquid (% by mol)] - 230" in the expression (1) is 183.

The reaction composition was supplied to a column filled with an ion exchange resin containing a carboxylic acid group as an exchange group (internal diameter: 800 mm, filling height: 1,600 mm) to adsorb and remove the catalyst (adsorption step (1)). The analyzed Na concentration in the reaction product at the ion exchange resin column outlet is 3 ppb by mass. At this time, the superficial velocity of the reaction composition in the adsorption step is 2.7 m/h and SV is 1.7 m$^3$/m$^3$-R.

The ion exchange resin column outlet liquid is supplied to the distillation step. The configuration of the distillation step is a configuration in which the column bottom outlet liquid of an unreacted raw material removal column as the first distillation column is supplied to a column similar to the above column filled with the ion exchange resin (adsorption step (2)), and is then supplied to a low-boiling point compound removal column as the second distillation column, and the column bottom outlet liquid of the second distillation column is supplied to a high-purity EMC purification column as the third distillation column. No blockage is caused over the entire production process, and continuous operation for 1,000 h or more is possible. The yield of ethyl methyl carbonate in the distillation step is 98% or more and 100% or less, and the yield of diethyl carbonate is also 98% or more and 100% or less.

[Example 4]

Using the apparatus for producing a carbonate having an ethyl group having a structure illustrated in Figure 5, carbonates having an ethyl group were produced as follows.

DMC, EtOH, and a 28% by mass solution of sodium methoxide in methanol were respectively supplied to a stirred tank reactor 1a having an internal diameter of 210 mm, a height of 300 mm, and a capacity of 7.3 L at a flow rate of 4,850 g/h, 1,450 g/h, and 1,125 mg/h, and reacted under conditions of 60°C and 103 kPaA, and 6,301 g/h of a reaction product was extracted from the stirred tank reactor 1a to obtain a reaction composition containing carbonates having an ethyl group. The concentration of sodium methoxide as the catalyst in the reaction composition was 50 ppm by mass, and was 68 mol ppm as the alkali metal concentration. With respect to the amount of each component in the reaction composition, MeOH was 751 g/h, EtOH was 370 g/h, DMC was 2,905 g/h, EMC was 2,055 g/h, and DEC was 220 g/h. The insoluble matter concentration in the reaction composition extracted from the stirred tank reactor 1a was 1 ppm by mass or less. The value of "0.14 × [reaction temperature (°C)] × [alcohol concentration in the reaction liquid (% by mol)] - 230" in the expression (1) was 80.

The reaction composition is supplied to a column filled with an ion exchange resin containing a sulfonic acid group as an exchange group (internal diameter: 30 mm, filling height: 500 mm) to adsorb and remove the catalyst (adsorption step (1)). At this time, the superficial velocity of the reaction composition in the adsorption step is 9.5 m/h and SV is 19 $m^3/m^3$-R. The analyzed Na concentration in the reaction product at the ion exchange resin column outlet was 60 ppb by mass.

The ion exchange resin column outlet liquid was filtered through a filter having a pore size of 3 $\mu$m and then supplied to the distillation step. The configuration of the distillation step was a configuration in which the column bottom outlet liquid of an unreacted raw material removal column as the first distillation column is supplied to a column similar to the above column filled with the ion exchange resin (adsorption step (2)), and is then supplied to a low-boiling point compound removal column as the second distillation column, and the column bottom outlet liquid of the second distillation column is supplied to a high-purity EMC purification column as the third distillation column.

As a result that the similar catalyst adsorption column was installed at the first distillation column bottom outlet, no blockage was caused over the entire production process, and continuous operation for 1,000 h or more was successfully conducted. The yield of ethyl methyl carbonate in the distillation step was 98% or more and 100% or less, and the yield of diethyl carbonate was also 98% or more and 100% or less.

[Example 5]

Using the apparatus for producing a carbonate having an ethyl group having a structure illustrated in Figure 6, carbonates having an ethyl group were produced as follows.

DMC, EtOH, and a 20% by mass solution of sodium ethoxide in ethanol were respectively supplied to a stirred tank reactor 1a having an internal diameter of 210 mm, a height of 300 mm, and a capacity of 7.3 L at a flow rate of 3,120 g/h, 1,400 g/h, and 1,780 mg/h, and reacted under conditions of 80°C and 103 kPaA, and 4,522 g/h of a reaction product was extracted from the stirred tank reactor 1a to obtain a reaction composition containing carbonates having an ethyl group. The concentration of sodium ethoxide as the catalyst in the reaction composition was 79 ppm by mass, and was 80 mol ppm as the alkali metal concentration. With respect to the amount of each component in the reaction composition, MeOH was 640 g/h, EtOH was 482 g/h, DMC was 1,520 g/h, EMC was 1,600 g/h, and DEC was 280 g/h. The insoluble matter concentration in the reaction composition extracted from the stirred tank reactor 1a was 10 ppm by mass, and the insoluble matter was iron oxide. The value of "0.14 × [reaction temperature (°C)] × [alcohol concentration in the reaction liquid (% by mol)] - 230" in the expression (1) was 294.

The reaction composition is supplied to a column filled with an ion exchange resin containing a carboxylic acid group as an exchange group (internal diameter: 80 mm, filling height: 500 mm) to adsorb and remove the catalyst (adsorption step (1)). At this time, the superficial velocity of the reaction composition in the adsorption step is 1.0 m/h and SV is 2.0 $m^3/m^3$-R. The analyzed Na concentration in the reaction product at the ion exchange resin column outlet was 3 ppb by mass.

The ion exchange resin column outlet liquid was supplied to the distillation step. The configuration of the distillation step was a configuration in which the column bottom outlet liquid of an unreacted raw material removal column as the first distillation column is supplied to a low-boiling point compound removal column as the second distillation column, and the column bottom outlet liquid of the second distillation column is supplied to a high-purity EMC purification column as the third distillation column.

As a result that the similar catalyst adsorption column was installed at the first distillation column bottom outlet, no blockage was caused over the entire production process, and continuous operation for 1,000 h or more was successfully conducted. The yield of ethyl methyl carbonate in the distillation step was 98% or more and 100% or less, and the yield of diethyl carbonate was also 98% or more and 100% or less.

[Example 6]

Using the apparatus for producing a carbonate having an ethyl group having a structure illustrated in Figure 5, carbonates having an ethyl group were produced as follows.

DMC, EtOH, and a 28% by mass solution of sodium methoxide in methanol were respectively supplied to a stirred tank reactor 1a having an internal diameter of 210 mm, a height of 300 mm, and a capacity of 7.3 L at a flow rate of 4,850 g/h, 1,450 g/h, and 1,125 mg/h, and reacted under conditions of 60°C and 103 kPaA, and 6,301 g/h of a reaction product was extracted from the stirred tank reactor 1a to obtain a reaction composition containing carbonates having an ethyl group. The concentration of sodium methoxide as the catalyst in the reaction composition was 50 ppm by mass, and was 68 mol ppm as the alkali metal concentration. With respect to the amount of each component in the reaction composition, MeOH was 751 g/h, EtOH was 370 g/h, DMC was 2,905 g/h, EMC was 2,055 g/h, and DEC was 220 g/h. The insoluble matter concentration in the reaction composition extracted from the stirred tank reactor 1a was 1 ppm by mass or less. The value of "0.14 × [reaction temperature (°C)] × [alcohol concentration in the reaction liquid (% by mol)] - 230" in the expression (1) was 80.

The reaction composition is supplied to a column filled with an ion exchange resin containing a sulfonic acid group as an exchange group (internal diameter: 15 mm, filling height: 50 mm) to adsorb and remove the catalyst (adsorption step (1)). At this time, the superficial velocity of the reaction composition in the adsorption step is 38 m/h and SV is 760 m3/m3-R. The analyzed Na concentration in the reaction product at the ion exchange resin column outlet was 1,200 ppb by mass.

The ion exchange resin column outlet liquid was filtered through a filter having a pore size of 3 μm and then supplied to the distillation step. The configuration of the distillation step was a configuration in which the column bottom outlet liquid of an unreacted raw material removal column as the first distillation column is supplied to a column similar to the above column filled with the ion exchange resin (adsorption step (2)), and is then supplied to a low-boiling point compound removal column as the second distillation column, and the column bottom outlet liquid of the second distillation column is supplied to a high-purity EMC purification column as the third distillation column.

As a result that the similar catalyst adsorption column was installed at the first distillation column bottom outlet, no blockage was caused over the entire production process, and continuous operation for 400 h or more was successfully conducted. The yield of ethyl methyl carbonate in the distillation step was 85% or more and 87% or less, and the yield of diethyl carbonate was 385% or more and 395% or less. The yield of diethyl carbonate exceeds 100%, and this is considered due to the conversion of dimethyl carbonate and ethyl methyl carbonate to diethyl carbonate by the remaining catalyst during distillation.

[Example 7]

Using the apparatus B for producing a carbonate having an ethyl group having a structure illustrated in Figure 3, carbonates having an ethyl group are produced as follows.

DMC, EtOH, and a 20% by mass solution of sodium ethoxide in ethanol are respectively supplied to a plug flow reactor 1b having six tubes with an internal diameter of 80 mm and a length of 10,000 mm (capacity: 0.3 m$^3$) at a flow rate of 156 kg/h, 70 kg/h, and 89 g/h, and reacted under the conditions of 70°C and 103 kPaA, and 226 kg/h of a reaction composition is extracted from the plug flow reactor 1b to obtain the reaction composition containing carbonates having an ethyl group. The concentration of sodium ethoxide as the catalyst in the reaction composition is 79 ppm by mass, and is 80 mol ppm as the alkali metal concentration. With respect to the amount of each component in the reaction composition, MeOH was 32 kg/h, EtOH was 24 kg/h, DMC was kg/h, EMC was 80 kg/h, and DEC was 14 kg/h. The insoluble matter concentration in the reaction composition extracted from the plug flow reactor 1b is 1 ppm by mass or less. The value of "0.14 × [reaction temperature (°C)] × [alcohol concentration in the reaction liquid (% by mol)] - 230" in the expression (1) was 228.

The reaction composition is supplied to a column filled with acidic activated alumina (internal diameter: 800 mm, filling height: 1,600 mm) to adsorb and remove the catalyst (adsorption step (1)). At this time, the superficial velocity of the reaction composition in the adsorption step is 0.05 m/h, and SV is 0.03 m$^3$/m$^3$-R. The analyzed Na concentration in the reaction product at the ion exchange resin column outlet is 410 ppb by mass.

The activated alumina column outlet liquid is supplied to the distillation step. The configuration of the distillation step is a configuration in which the column bottom outlet liquid of an unreacted raw material removal column as the first distillation column is supplied to a low-boiling point compound removal column as the second distillation column, and the column bottom outlet liquid of the second distillation column is supplied to a high-purity EMC purification column as the third distillation column. No blockage is caused over the entire production process, and continuous operation for 400 h or more is possible. The yield of ethyl methyl carbonate in the distillation step is 95% or more and 99% or less, and the yield of diethyl carbonate is 115% or more and 125% or less.

[Comparative Example 1]

Using a production apparatus obtained by removing the column filled with the ion exchange resin from the apparatus A for producing a carbonate having an ethyl group having a structure illustrated in Figure 2, carbonates having an ethyl group are produced as follows.

DMC, EtOH, and a 28% by mass sodium methoxide solution are respectively supplied to a stirred tank reactor 1a having a capacity of 6 m$^3$ at a flow rate of 970 kg/h, 290 kg/h, and 225 g/h, and reacted at 60°C and 103 kPaG, and 1,260 kg/h of a reaction composition is extracted from the stirred tank reactor 1a to obtain carbonates having an ethyl group. The concentration of sodium methoxide as the catalyst in the reaction composition is 50 ppm by mass, and is 68 mol ppm as the alkali metal concentration. Since the condition of the catalyst concentration in the reaction product satisfies the expression (1) in the present reaction, the insoluble matter concentration in the reaction product is 1 ppm by mass or less. The value of "0.14 × [reaction temperature (°C)] × [alcohol concentration in the reaction liquid (% by mol)] - 230" in the expression (1) is 56.

The obtained reaction composition is supplied to the distillation step. The configuration of the distillation step is a configuration in which the column bottom outlet liquid of an unreacted raw material removal column as the first distillation column is supplied to a low-boiling point compound removal column as the second distillation column, and the column bottom outlet liquid of the second distillation column is supplied to a high-purity EMC purification column as the third distillation column. Since blockage is caused in the first distillation column, operation for 100 h or more is impossible. The yield of ethyl methyl carbonate in the distillation step is 57% or more and 62% or less, and the yield of diethyl carbonate is 390% or more and 410% or less.

[Comparative Example 2]

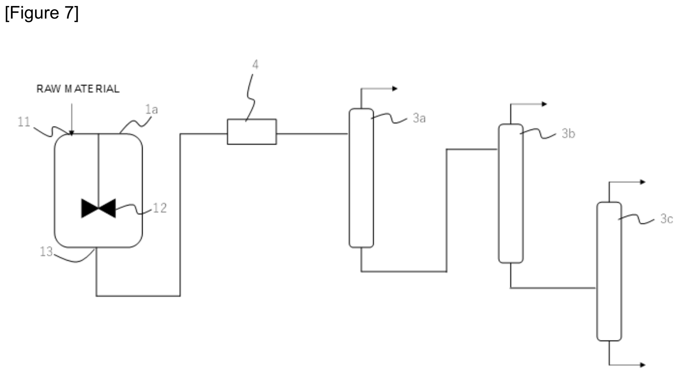

Using the apparatus for producing a carbonate having an ethyl group having a structure illustrated in Figure 7, carbonates having an ethyl group were produced as follows.

DMC, EtOH, and a 28% by mass solution of sodium methoxide in methanol were respectively supplied to a stirred tank reactor 1a having an internal diameter of 210 mm, a height of 300 mm, and a capacity of 7.3 L at a flow rate of 4,850 g/h, 1,450 g/h, and 7,875 mg/h, and reacted under conditions of 60°C and 103 kPaA, and 6,308 g/h of a reaction product was extracted from the stirred tank reactor 1a to obtain a reaction composition containing carbonates having an ethyl group. The concentration of sodium methoxide as the catalyst in the reaction composition was 350 ppm by mass, and was 1,707 mol ppm as the alkali metal concentration. With respect to the amount of each component in the reaction composition, MeOH was 770 g/h, EtOH was 354 g/h, DMC was 2,937 g/h, EMC was 1,943 g/h, and DEC was 303 g/h. The insoluble matter concentration in the reaction composition extracted from the stirred tank reactor 1a was 240 ppm by mass. The value of "0.14 × [reaction temperature (°C)] × [alcohol concentration in the reaction liquid (% by mol)] - 230" in the expression (1) was 80.

The reaction composition is supplied to a filter press machine, and the insoluble matter is removed. The analyzed Na concentration in the reaction product at the filter press machine outlet was 46 ppm by mass.

The filter press machine outlet liquid is supplied to the distillation step. The configuration of the distillation step is a configuration in which the column bottom outlet liquid of an unreacted raw material removal column as the first distillation column is supplied to a low-boiling point compound removal column as the second distillation column, and the column bottom outlet liquid of the second distillation column is supplied to a high-purity EMC purification column as the third distillation column. Since blockage was caused in the first distillation column, operation for 100 h or more was impossible. The yield of ethyl methyl carbonate in the distillation step was 65% or more and 70% or less, and the yield of diethyl carbonate was 315% or more and 325% or less.

[Table 1-1]

| Table 1 (1/3) | | | Example 1 | Example 2 | Example 3 |
|---|---|---|---|---|---|
| Reaction step | | Supply flow rate | DMC 970kg/h<br>EtOH 290kg/h<br>Sodium methoxide solution 225g/h (28% solution of sodium methoxide in methanol) | DMC 156kg/h<br>EtOH 70kg/h<br>Sodium ethoxide solution 89g/h (20% solution of sodium ethoxide in ethanol) | DMC 970kg/h<br>EtOH 290kg/h<br><br>Sodium methoxide solution 1,100g/h (28% solution of sodium methoxide in methanol) |
| | | Catalyst concentration | 50ppm by mass (68 mol ppm) | 79ppm by mass (80 mol ppm) | 224ppm by mass (334mol ppm) |
| | | Reactor | Stirred tank reactor | Plug flow reactor | Stirred tank reactor |
| | | Reaction conditions | 60°C/103kPaA | 70°C/103kPaA | 80°C/200kPaA |
| | | Extraction flow rate | 1,260kg/h | 226 kg/h | 1,261kg/h |
| | | Insoluble matter concentration | $\leq$ 1ppm by mass | $\leq$1ppm by mass | The reaction composition was supplied to a filter, and the insoluble matter in the reaction product after filtration was 1 ppm by wt or less.<br>The insoluble matter concentration in the reaction composition before filtration was 83 ppm by wt. |
| | | | 8.4 | | |
| Step subsequent to reaction step (catalyst adsorption step) | | Catalyst adsorbent | Acidic cation exchange resin (sulfonic acid group) | Acidic cation exchange resin (carboxylic acid group) | Acidic cation exchange resin (carboxylic acid group) |
| | | Superficial velocity/SV | 2.7m/h/1.7m3/m3-R | 0.05m/h /0.03m3/m3-R | 2.7m/h/1.7m3/m3-R |
| | | Alkali metal concentration at step outlet | 3ppb by mass (Na concentration) | 1ppb by mass (Na concentration) | 3ppb by mass (Na concentration) |

(continued)

| Table 1 (1/3) | | | Example 1 | Example 2 | Example 3 |
|---|---|---|---|---|---|
| Distillation step | | | First column: unreacted raw material removal column<br><br>Second column: low-boiling point compound removal column<br><br>Third column: high-purity EMC purification column | First column: unreacted raw material removal column<br>Second column: low-boiling point compound removal column<br>Third column: high-purity EMC purification column | First column: unreacted raw material removal column<br><br>Second column: low-boiling point compound removal column<br><br>Third column: high-purity EMC purification column |
| | EMC yield in distillation step | | 98% or more and 100% or less | 98% or more and 100% or less | 98% or more and 100% or less |
| | DEC yield in distillation step | | 98% or more and 100% or less | 98% or more and 100% or less | 98% or more and 100% or less |
| | Continuous operation time | | 1,000h or more | 1,000h or more | 1,000h or more |

[Table 1-2]

| Table 1 (2/3) | | | Example 4 | Example 5 | Example 6 |
|---|---|---|---|---|---|
| Reaction step | | Supply flow rate | DMC 4,850g/h<br>EtOH 1,450g/h<br>Sodium methoxide solution 1,125mg/h (28% solution of sodium methoxide in methanol) | DMC 3,120g/h<br>EtOH 1,400g/h<br>Sodium ethoxide solution 1,780mg/h (28% solution of sodium methoxide in methanol) | DMC 4,850g/h<br>EtOH 1,450g/h<br>Sodium methoxide solution 1,125mg/h (28% solution of sodium methoxide in methanol) |
| | | Catalyst concentration | 50ppm by mass (68mol ppm) | 79ppm by mass (80mol ppm) | 50ppm by mass (68mol ppm) |
| | | Reactor | Stirred tank reactor | Stirred tank reactor | Stirred tank reactor |
| | | Reaction conditions | 60°C/103kPaA | 80°C/103kPaA | 60°C/103kPaA |
| | | Extraction flow rate | 6,301g/h | 4,522g/h | 6,301g/h |
| | | Insoluble matter concentration | ≤1ppm by mass | 10ppm by mass (iron oxide) | ≤1ppm by mass |

(continued)

| Table 1 (2/3) | | | | | |
|---|---|---|---|---|---|
| | | | Example 4 | Example 5 | Example 6 |
| Step subsequent to reaction step (catalyst adsorption step) | | Catalyst adsorbent | Acidic cation exchange resin (sulfonic acid group) | Acidic cation exchange resin (carboxylic acid group) | Acidic cation exchange resin (sulfonic acid group) |
| | | Superficial velocity/SV | 9.5m/h/19.0m3/m3-R | 1.0m/h/2.0m3/m3-R | 38m/h/760m3/m3-R |
| | | Alkali metal concentration at step outlet | 60ppb by mass (Na concentration) | 3ppb by mass (Na concentration) | 1,200ppb by mass (Na concentration) |
| Distillation step | | | First column: unreacted raw material removal column / Second column: low-boiling point compound removal column / Third column: high-purity EMC purification column | First column: unreacted raw material removal column / Second column: low-boiling point compound removal column / Third column: high-purity EMC purification column | First column: unreacted raw material removal column / Second column: low-boiling point compound removal column / Third column: high-purity EMC purification column |
| | | EMC yield in distillation step | 98% or more and 100% or less | 98% or more and 100% or less | 85% or more and 87% or less |
| | | DEC yield in distillation step | 98% or more and 100% or less | 98% or more and 100% or less | 385% or more and 395% or less |
| | | Continuous operation time | 1,000h or more | 1,000h or more | 800h or more |

[Table 1-3]

| Table 1 (3/3) | | | | | |
|---|---|---|---|---|---|
| | | | Example 7 | Comparative Example 1 | Comparative Example 2 |
| Reaction step | | Supply flow rate | DMC 156kg/h / EtOH 70kg/h / Sodium ethoxide solution 89g/h (20% solution of sodium ethoxide in ethanol) | DMC 970kg/h / EtOH 290kg/h / Sodium methoxide solution 225g/h (28% solution of sodium methoxide in methanol) | DMC 4,850g/h / EtOH 1,450g/h / Sodium methoxide solution 7,875mg/h (28% solution of sodium methoxide in methanol) |
| | | Catalyst concentration | 79ppm by mass (80mol ppm) | 50wtppm (68mol ppm) | 350wtppm (1,707mol ppm) |
| | | Reactor | Plug flow reactor | Stirred tank reactor | Stirred tank reactor |
| | | Reaction conditions | 70°C/103kPaA | 60°C/103kPaA | 60°C/103kPaA |
| | | Extraction flow rate | 226 kg/h | 1,260kg/h | 6,308g/h |
| | | Insoluble matter concentration | ≤1ppm by mass | ≤1 ppm by mass | 240ppm by mass |

(continued)

| Table 1 (3/3) | | | | |
|---|---|---|---|---|
| | | Example 7 | Comparative Example 1 | Comparative Example 2 |
| Step subsequent to reaction step (catalyst adsorption step) | Catalyst adsorbent | Acidic activated alumina | Absence | Absence |
| | Superficial velocity/SV | 0.05m/h /0.03m3/m3-R | | |
| | Alkali metal concentration at step outlet | 410ppb by mass (Na concentration) | 21ppm by mass (Na concentration) | 46ppm by mass (Na concentration) |
| Distillation step | | First column: unreacted raw material removal column Second column: low-boiling point compound removal column Third column: high-purity EMC purification column | First column: unreacted raw material removal column Second column: low-boiling point compound removal column Third column: high-purity EMC purification column | First column: unreacted raw material removal column Second column: low-boiling point compound removal column Third column: high-purity EMC purification column |
| | EMC yield in distillation step | 97% or more and 99% or less | 57% or more and 59% or less | 65% or more and 70% or less |
| | DEC yield in distillation step | 115% or more and 125% or less | 397% or more and 399% or less | 315% or more and 325% or less |
| | Continuous operation time | 800h or more | 100h or less | 100h or less |

**Industrial Applicability**

According to the method for producing a carbonate having an ethyl group of the present invention, substances such as ethyl methyl carbonate and diethyl carbonate used as the electrolyte solution for lithium-ion secondary batteries can be efficiently produced.

**Reference Signs List**

1: Reaction apparatus
1a: Stirred tank reactor
11: Raw material supply part
12: Stirring apparatus
13: Extraction part
1b: Plug flow reactor
2: Catalyst adsorption apparatus
3: Distillation apparatus
3a: Unreacted raw material removal distillation column
3b: Low-boiling point compound removal distillation column
3c: High-purity ethyl methyl carbonate purification distillation column

**Claims**

1. A method for producing a carbonate having an ethyl group, the method comprising:

   a reaction step of subjecting dimethyl carbonate and ethanol to transesterification reaction in a reactor in the presence of a catalyst containing an alkali metal compound to obtain a reaction composition; and
   a distillation step of distilling the reaction composition in a distillation column,
   wherein the method comprises an adsorption step of adsorbing the alkali metal compound in the reaction composition by an adsorbent before the distillation step or during the distillation step.

2. The method for producing the carbonate having the ethyl group according to claim 1, wherein an insoluble matter concentration in the reaction composition in the reaction step is set to 50 ppm by mass or less.

3. The method for producing the carbonate having the ethyl group according to claim 1, wherein an alkali metal concentration in the reaction composition treated in the adsorption step is 500 ppb by mass or less.

4. The method for producing the carbonate having the ethyl group according to claim 1, wherein the adsorbent in the adsorption step is a solid adsorbent.

5. The method for producing the carbonate having the ethyl group according to claim 1, wherein the adsorbent in the adsorption step is an acidic cation exchange resin.

6. The method for producing the carbonate having the ethyl group according to claim 5, wherein the acidic cation exchange resin comprises a sulfonic acid group or a carboxylic acid group.

7. The method for producing the carbonate having the ethyl group according to claim 1, wherein a concentration of the catalyst supplied to the reactor in the reaction step satisfies a condition of the expression (1):

   [alkali metal concentration in the reaction composition (mol ppm)] $\leq$ 0.14 $\times$ [reaction temperature (°C)] $\times$ [alcohol concentration in reaction liquid (% by mol)] - 230     expression (1).

8. The method for producing the carbonate having the ethyl group according to claim 1, further comprising a filtration step of filtering the reaction composition after the adsorption step.

9. The method for producing the carbonate having the ethyl group according to claim 1, wherein a reaction temperature in the reaction step is 30°C or more and 110°C or less.

10. The method for producing the carbonate having the ethyl group according to claim 1, wherein the reactor used in the reaction step is a stirred tank reactor or a plug flow reactor.

11. The method for producing the carbonate having the ethyl group according to claim 1, wherein an adsorption column filled with the adsorbent is used in the adsorption step, and a superficial velocity of the adsorption column is 0.05 to 15 m/h.

12. The method for producing the carbonate having the ethyl group according to claim 1, wherein a relationship between a flow rate of the adsorption column and an amount of the adsorbent filled, SV = [flow rate $m^3$/h]/[amount of the adsorbent filled $m^3$-R] is 0.02 or more and 30 or less in the adsorption step.

13. The method for producing the carbonate having the ethyl group according to claim 1, wherein the distillation step comprises at least one selected from the group consisting of an unreacted raw material removal step, a low-boiling point compound removal step, a high-purity ethyl methyl carbonate purification step, and a high-purity diethyl carbonate purification step.

14. The method for producing the carbonate having the ethyl group according to claim 13, wherein the distillation step comprises at least three selected from the group.

15. The method for producing the carbonate having the ethyl group according to claim 1, wherein the distillation step comprises:

an unreacted raw material removal step,
a low-boiling point compound removal step, and
a high-purity ethyl methyl carbonate purification step or a high-purity diethyl carbonate purification step.

16. An apparatus for producing a carbonate having an ethyl group, the apparatus comprising:

a reaction apparatus for subjecting dimethyl carbonate and ethanol to transesterification reaction in a reactor in the presence of a catalyst containing an alkali metal compound to obtain a reaction composition;
a catalyst adsorption apparatus for adsorbing the catalyst from the reaction composition by an adsorbent; and
a distillation apparatus for distilling the reaction composition.

17. The apparatus for producing the carbonate having the ethyl group according to claim 16, wherein the adsorbent is a solid adsorbent.

18. The apparatus for producing the carbonate having the ethyl group according to claim 16, wherein the adsorbent is an acidic cation exchange resin.

19. The apparatus for producing the carbonate having the ethyl group according to claim 18, wherein the acidic cation exchange resin comprises a sulfonic acid group or a carboxylic acid group.

20. The apparatus for producing the carbonate having the ethyl group according to claim 16, wherein the catalyst adsorption apparatus is an adsorption column filled with the adsorbent.

21. The apparatus for producing the carbonate having the ethyl group according to claim 16, further comprising a filter for filtering the reaction composition downstream the catalyst adsorption apparatus.

22. The apparatus for producing the carbonate having the ethyl group according to claim 16, wherein the reaction apparatus is a stirred tank reactor or a plug flow reactor.

23. The apparatus for producing the carbonate having the ethyl group according to claim 16, wherein the distillation apparatus comprises at least one selected from the group consisting of an unreacted raw material removal distillation column, a low-boiling point compound removal distillation column, a high-purity ethyl methyl carbonate purification distillation column, and a high-purity diethyl carbonate purification distillation column.

24. The apparatus for producing the carbonate having the ethyl group according to claim 23, wherein the distillation apparatus contains at least three selected from the group.

25. The apparatus for producing the carbonate having the ethyl group according to claim 16, wherein the distillation apparatus comprises:

an unreacted raw material removal distillation column,
a low-boiling point compound removal distillation column, and
a high-purity ethyl methyl carbonate purification distillation column or a high-purity diethyl carbonate purification distillation column.

[Figure 1]

[Figure 2]

[Figure 3]

[Figure 4]

[Figure 5]

[Figure 6]

[Figure 7]

## INTERNATIONAL SEARCH REPORT

| International application No. |
|---|
| **PCT/JP2024/020877** |

| A. | CLASSIFICATION OF SUBJECT MATTER |
|---|---|

*C07C 68/08*(2006.01)i; *C07B 61/00*(2006.01)i; *C07C 68/06*(2020.01)i; *C07C 68/065*(2020.01)i; *C07C 69/96*(2006.01)i
FI: C07C68/08; C07C69/96 Z; C07C68/06; C07B61/00 300; C07C68/065

According to International Patent Classification (IPC) or to both national classification and IPC

| B. | FIELDS SEARCHED |
|---|---|

Minimum documentation searched (classification system followed by classification symbols)

C07C68/08; C07B61/00; C07C68/06; C07C68/065; C07C69/96

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN); CASREACT(STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2012-510975 A (SHELL INTERNATIONALE RESEARCH MAATSCHAPPIJ B.V.) 17 May 2012 (2012-05-17) claims, paragraphs [0015], [0021], [0027], [0006], examples, etc. | 1-25 |
| Y | WO 2022/114576 A1 (LOTTE CHEMICAL CORPORATION) 02 June 2022 (2022-06-02) claims, paragraphs [0012]-[0014], examples, etc. | 1-25 |
| Y | KR 10-2023-0080683 A (LOTTE CHEMICAL CORPORATION) 07 June 2023 (2023-06-07) claims, examples, etc. | 1-25 |

☐ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 August 2024** | **20 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/020877**

| Patent document cited in search report | | | Publication date (day/month/year) | Patent family member(s) | | | Publication date (day/month/year) |
|---|---|---|---|---|---|---|---|
| JP | 2012-510975 | A | 17 May 2012 | WO | 2010/063780 | A1 | |
| | | | | claims, p. 5, lines 3-20, p. 2, lines 20-32, p. 7, lines 4-27, examples | | | |
| | | | | CN | 102177130 | A | |
| WO | 2022/114576 | A1 | 02 June 2022 | US | 2024/0002330 | A1 | |
| | | | | claims, paragraphs [0011]-[0012], examples | | | |
| | | | | JP | 2023-552122 | A | |
| | | | | EP | 4253364 | A1 | |
| | | | | KR | 10-2022-0073559 | A | |
| | | | | CN | 116745259 | A | |
| KR | 10-2023-0080683 | A | 07 June 2023 | WO | 2023/101298 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022114592 A

- WO 2022114576 A